# EUROPEAN PATENT SPECIFICATION

(11) **EP 1 706 394 B1**
(45) Date of publication and mention of the grant of the patent: **17.12.2014**
(21) Application number: 04811264.3
(22) Date of filing: 12.11.2004
(51) Int. Cl.: C07D 307/87

(54) **PREPARATION OF ESCITALOPRAM**
HERSTELLUNG VON ESCITALOPRAM
PRÉPARATION D'ESCITALOPRAME

(30) Priority: 12.11.2003 IN CH09242003; 22.04.2004 IN CH03702004; 04.08.2004 US 598725 P
(43) Date of publication of application: 04.10.2006
(73) Proprietor: Dr. Reddy's Laboratories, Inc., Bridgewater, NJ 08807 (US); Dr. Reddy's Laboratories Ltd., Hyderabad 500 016 (IN)
(72) Inventor: SUNDARAM, Venkataraman, Plot No 141, Flat No. 202, Andhra Pradesh, Hyderabad 500 018 (IN); MATHAD, Vijayavitthai Thippannachar, Nashik 422 202, Maharashira (IN); VENKAVALA, Pravinachandra Jayanthilal, Andhra Pradesh, Hyderabad 500 072 (IN); ELATI, Chandrashekar Ravirama, Andhra Pradesh, Hyderabad 500 072 (IN); KOLLA, Naveenkumar, Andhra Pradesh, Hyderabad 500 072 (IN); GOVINDAN, Shanmugam, Chennai 600 078, Tamil Nadu (IN); CHALAMALA, Subrahmanyeshwara Rao, Andhra Pradesh, Hyderabad 500 072 (IN); GANGULA, Srinivas, Andhra Pradesh, Hyderabad 500 072 (IN)
(74) Representative: Bates, Philip Ian
(86) International application number: PCT/US2004/038490
(87) International publication number: WO 2005/047274

(56) References cited:
- EP-A- 0 347 066
- EP-A1- 0 169 302
- EP-A1- 0 768 309
- EP-A2- 0 577 171
- WO-A-03/051861
- WO-A1-01/68629
- WO-A2-01/43525
- WO-A2-02/16202
- US-A- 5 280 122
- US-A1- 2003 153 750
- US-B1- 6 458 975
- US-B1- 6 781 003
- DATABASE CROSSFIRE BEILSTEIN BEILSTEIN INSTITUT ZUR FOERDERUNG DER CHEMISCHEN WISSENSCHAFTEN, FRANKFURT AM MAIN, DE; XP002494872 Database accession no. 6547529 & EUROPEAN JOURNAL OF MEDICINAL CHEMISTRY, vol. 12, 1977, pages 289-295,
- CHANDRASHEKAR R. ELATI ET AL: ', 11 , 289-292]' ORGANIC PROCESS RESEARCH & DEVELOPMENT vol. 13, no. 1, 16 January 2009, pages 34 - 37, XP055028207 DOI: 10.1021/op8002079 ISSN: 1083-6160
- BIGLER A J ET AL: "QUANTITATIVE STRUCTURE-ACTIVITY RELATIONSHIPS IN A SERIES OF SELECTIVE 5-HT UPTAKE INHIBITORS", EUROPEAN JOURNAL OF MEDICINAL CHEMISTRY, EDITIONS SCIENTIFIQUE ELSEVIER, PARIS, FR, vol. 12, no. 3, 1 January 1977 (1977-01-01), page 289/290, XP002941313, ISSN: 0223-5234
- PARK HYEUNG GEUN ET AL: "Synthesis of 2-substituted-pyrrolidinethiourea derivatives and their antagonist effect on vanilloid receptor.", BIOORGANIC & MEDICINAL CHEMISTRY LETTERS 20 JAN 2003, vol. 13, no. 2, 20 January 2003 (2003-01-20), pages 197-200, ISSN: 0960-894X
- BAO XING-FENG ET AL: "structural characterization and chemical modification of a glucan from spores of ganoderma lucidum", , vol. 12, no. 11, 2001, pages 967-970,
- GOBBINI M ET AL: "Digitalis-like compounds: synthesis and biological evaluation of 3beta-(aminoalkylthio) derivatives", BIOORGANIC & MEDICINAL CHEMISTRY LETTERS, PERGAMON, AMSTERDAM, NL, vol. 7, no. 4, 18 February 1997 (1997-02-18), pages 469-472, XP004136048, ISSN: 0960-894X, DOI: 10.1016/S0960-894X(97)00039-5
- S. D. TURK ET AL: "Addition of Hydrogen Sulfide to Unsaturated Amines", THE JOURNAL OF ORGANIC CHEMISTRY, vol. 27, no. 8, 1 August 1962 (1962-08-01) , pages 2846-2853, XP055109526, ISSN: 0022-3263, DOI: 10.1021/jo01055a030
- PESTI J: "Editorial: Scientific disputes and how OPRD handles them", ORGANIC PROCESS RESEARCH AND DEVELOPMENT, CAMBRIDGE, GB, [Online] vol. 13, no. 1, 1 January 2009 (2009-01-01), page 22, XP003029106, DOI: 10.1021/OP800283G Retrieved from the Internet: URL:http://pubs.acs.org/doi/abs/10.1021/op 800283g> [retrieved on 2008-11-21]
- ROBERT JAMES DANCER ET AL: "Response to the comments by Elati et al. in response to our article examining one of their previous articles", ORGANIC PROCESS RESEARCH AND DEVELOPMENT, CAMBRIDGE, GB, [Online] vol. 13, no. 1, 1 January 2009 (2009-01-01), pages 38-43, XP003029105, DOI: 10.1021/OP800252W Retrieved from the Internet: URL:http://pubs.acs.org/doi/abs/10.1021/op 800252w> [retrieved on 2008-11-21]
- DANCER R J ET AL: "Attempted resolution of citalopram using (-)-O,O-Di-p-toluoyl-(R,R)-tartaric acid, and reflections on an alkylation reaction; Comment on an article by Elatie et al", ORGANIC PROCESS RESEARCH AND DEVELOPMENT, CAMBRIDGE, GB, vol. 13, 1 January 2009 (2009-01-01), pages 23-33, XP003029103,
- CHANDRASHEKAR R ELATI ET AL: "Substrate modification approach to achieve efficient resolution: Didesmethylcitalopram: A key intermediate for escitalopram (Additions & Corrections)", ORGANIC PROCESS RESEARCH AND DEVELOPMENT, CAMBRIDGE, GB, [Online] vol. 11, no. 4, 1 January 2007 (2007-01-01), page 780, XP003029102, DOI: 10.1021/OP700124V Retrieved from the Internet: URL:http://pubs.acs.org/doi/abs/10.1021/op 700124v> [retrieved on 2007-06-27]
- ELATI C R ET AL: "Substrate Modification Approach to Achieve Efficient resolution: Didesmethylcitalopram: a Key Intermediate for Escitalopram", TETRAHEDRON LETTERS, PERGAMON, GB, vol. 11, no. 2, 1 January 2007 (2007-01-01), pages 289-292, XP002491129, ISSN: 0040-4039

## Description

### INTRODUCTION TO THE INVENTION

The present invention relates to a process for the preparation of the drug compound escitalopram, which is chemically known as (+)-1-(3-dimethylaminopropyl)-1-(4-fluorophenyl)-1,3-dihydroisobenzofuran-5-carbonitrile, being the S-enantiomer of citalopram, and is represented by the following formula:

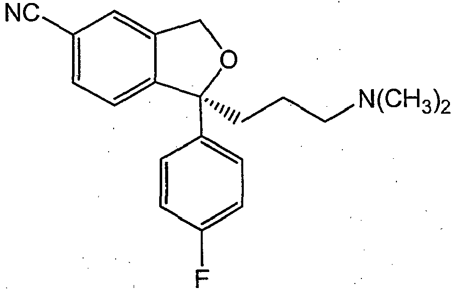

Escitalopram has been disclosed in U.S. Patent 4,943,590 that corresponds to European Patent 0 347 066 B1. This patent describes two processes for the preparation of escitalopram. Both of the processes utilize the racemic diol having the following formula, as a starting material:

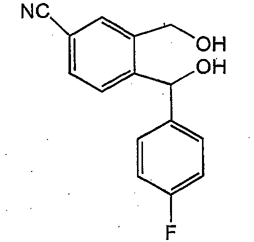

According to the first process, the diol is reacted with an enantiomerically pure acid derivative, such as (+) or (-)-*α*-methoxy-*α*-triflouromethylphenylacetyl chloride to form a mixture of diastereomeric esters, which are separated by HPLC or fractional crystallization, and then the ester with the correct stereochemistry is converted into escitalopram by cyclization.

According to the second process, the diol is separated by treating with an enantiomerically pure acid such as (+)-di-p-toluoyl tartaric acid, followed by separating the required isomer by crystallization. Cyclization yields escitalopram. Both routes have been found to be economically and environmentally undesirable due to low product yields.

International Publication WO 03/006449 discloses a process for the preparation of escitalopram, involving chromatographic separation of the enantiomers of citalopram and Intermediate using a chiral stationary phase, which process is not commercially feasible and not economical.

International Publication WO 03/087081 describes a process for the preparation of escitalopram via (4-bromo-2-(hydroxymethyl) phenyl)-(4-flouro phenyl) methanol, where the racemic diol is converted to an enantiomerically enriched form by first converting the diol into monoester intermediate and then reacting the monoester intermediate with an optically active acid to form a diastereomeric salt. This salt is then crystallized to obtain an enantiomerically enriched S-isomer whereupon the monoester intermediate is further converted to escitalopram through suitable chemical conversions. The major drawbacks of the described process are low yields and usage of the hazardous material copper cyanide, plus a lengthy process of production.

International Publication WO 03/051861 describes the separation of racemic Br-citalopram to the corresponding S-Br-citalopram by fractional crystallization of a diastereomeric salt of Br-citalopram, followed by hydrolysis and cyanation to get the escitalopram. The major drawback of this process is the use of a cyanide in the presence of palladium or nickel catalyst for the conversion of bromo to cyano groups, which is industrially not desirable due to safety concerns.

This patent also describes the separation of the bromodiol intermediate by chromatography using a chiral stationary phase, which is industrially not feasible to practice at the plant level.

A need remains for a cost effective, safe, and industrially feasible route to synthesize escitalopram and an invention made toward this goal is described below.

WO01/43525A2 discloses processes for the preparation of didesmethylcitalopram at Page 4, Line 4-6; Page 5, Line 10-15; Page 7, Line 5-13 and Example 9 (Page No. 17, Line No. 29-36).

WO01/68629A1 discloses a process for the preparation of didesmethylcitalopram at Page 5, Line 10-14.

### SUMMARY OF THE INVENTION

The invention pertains to a process for preparing escitalopram comprising the steps of:
(a) reacting 5-cyano-1-(4-fluorophenyl)-1-aminopropyl-1,3-dihydroisobenzofuran having the structure: 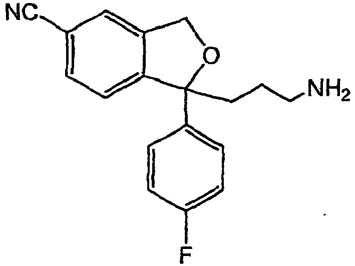 with an enantiomerically pure acid;
(b) hydrolyzing a product from (a) using a base;
(c) methylating a product recovered from (b); and
(d) recovering escitalopram.

One process for preparing escitalopram involves first preparing didesmethyl citalopram of the following formula, which is a useful intermediate in the preparation of escitalopram, and includes a dynamic resolution of didesmethyl citalopram followed by methylation to get the desired escitalopram.

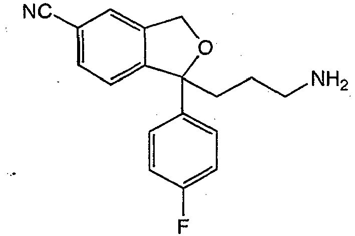

Another process for the production of citalopram involves condensation of isobenzofuran of Formula (VI) with chloropropyl amine of Formula (VII) to afford a compound of Formula (II), which is further subjected to resolution followed by methylation to afford citalopram of Formula-I, according to the following Scheme I.

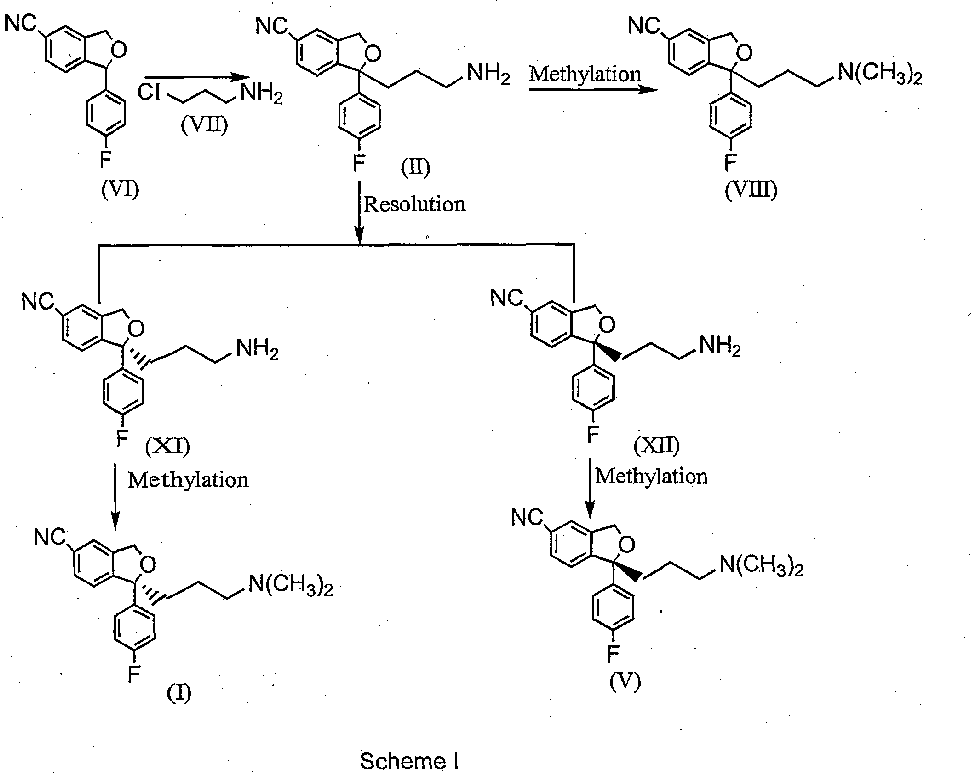

Another process for the production of escitalopram of the disclosure includes preparation of racemic 1-(3-aminopropyl)-1-(4-fluorophenyl)-1,3-dihydro-5-isobenzofuran carbonitrile of Formula (II) by condensation of the isobenzofuran of Formula (VI) with chloropropyl amine of Formula (VII) described in the following Scheme II.

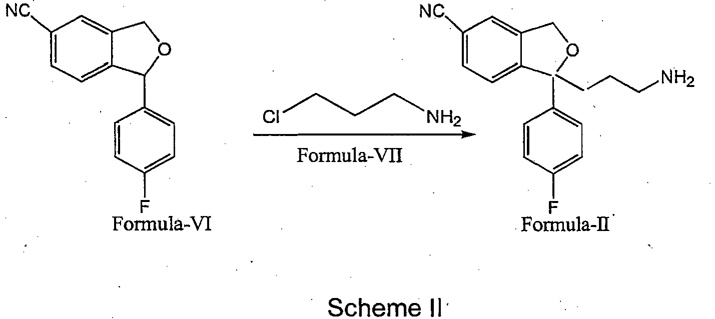

Still another process for the preparation of escitalopram of the disclosure proceeds through the resolution of the racemic 1-(3-amino propyl)-1-(4-fluorophenyl)-1,3-dihydro-5-isobenzofurancarbonitrile of Formula II to its corresponding (+)-1-(3-aminopropyl)-1-(4-fluorophenyl)-1,3-dihydro-5-isobenzofurancarbonitrile of Formula XI and (-)-1-(3-amino propyl)-1-(4-fluorophenyl)-1,3-dihydro-5-isobenzofuran carbonitrile of Formula XII, through the dynamic diastereomeric salt formation of the amine with chiral acids followed by their hydrolysis as shown in the below Scheme III

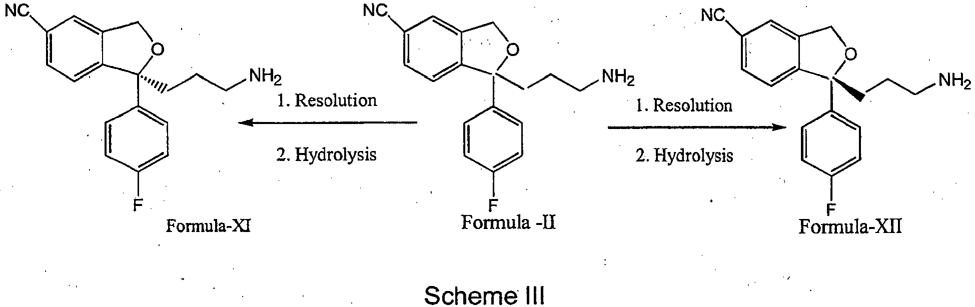

Another process for the preparation of S-citalopram and R-citalopram is by methylation of the compounds of Formula XI and Formula XII respectively, as described in Scheme IV.

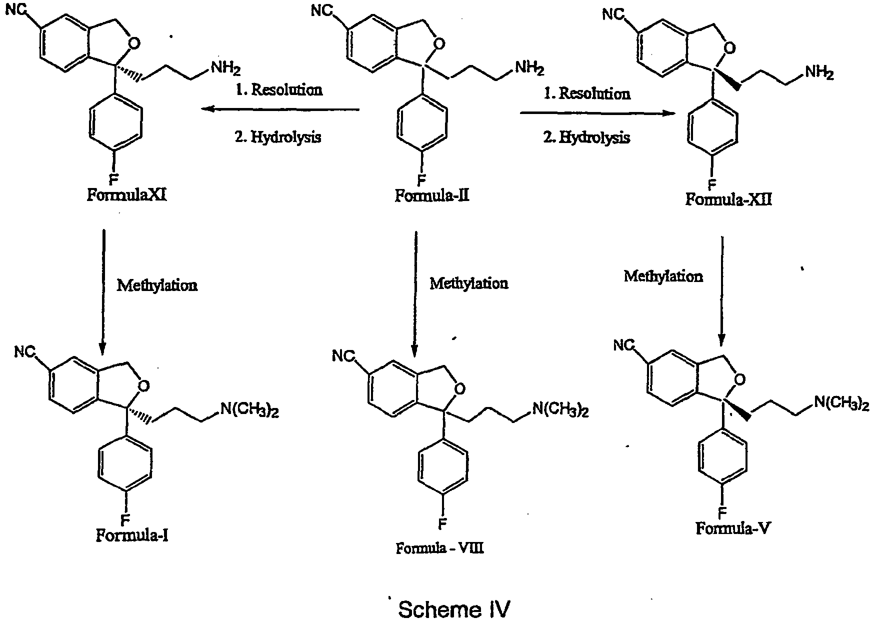

A further process for the preparation of citalopram by methylation of the amino compound having Formula II in a high yield and purity is as shown in Scheme IV.

Escitalopram can also be prepared from citalopram by diastereomeric salt formation followed by separation and hydrolysis.

### DETAILED DESCRIPTION

Referring to the preceding Scheme I, one process for the preparation of escitalopram comprises the alkylation of 5-cyano-1-(4-flurophenyl)-1,3-dihydroisobenzofuran of Formula VI with a compound of formula VII in a suitable base and solvent, to afford racemic 1-(3-aminopropyl)-1-(4-flourophenyl)-1,3 dihydro-5-isobenzofurancarbonitrile of Formula II. The racemic product is subjected to resolution with a suitable chiral acid in a suitable solvent system to obtain a diastereomeric salt of the amine. Hydrolysis of the obtained diastereomeric salt, followed by methylation of the compounds of Formula XI or Formula XII affords escitalopram of Formula I or R-citalopram of Formula (V).

Didesmethylcitalopram of Formula II can be prepared by:
(i) heating a solution of a base selected from: LDA (lithium diisopropyl amine); NaH; n-BuLi; and metal oxides such as NaOMe, KOMe, LiOMe, NaO-*t*-Bu, KO-*t*-Bu and LiO-*t*-Bu: more preferably NaH, or KO-*t*-Bu or LDA, more preferably in KO-*t*-Bu in an aprotic solvent selected from the list of DMSO, THF (tetrahydrofuran), DMF (dimethyl formide), NMP (N-methyl pyrrolidone), ethers; such as diethyl ether, methyl tert-butyl ether, ketones such as acetone, methyl ethyl ketone, or methyl isobutyl ketone, hydrocarbons; such as toluene, benzene, cyclohexane or alkanes, and mixtures thereof; preferably dimethyl sulfoxide or acetone or toluene, more preferably dimethyl sulfoxide, in a anhydrous condition under nitrogen atmosphere, at a temperature about 50-120°C, preferably about 60-65°C, for at least an hour;
(ii) cooling the reaction mixture of step (1) to temperature between about 10-50°C, preferably to a temperature of about 30-35°C;
(iii) dissolving 5-cyano-1-(4-fluoro phenyl)-1,3-dihydrobenzofuran of formula VI in a suitable aprotic solvent such as dimethyl sulfoxide (DMSO), THF (tetrahydrofuran), DMF (dimethyl formide), NMP (N-methyl pyrrolidone), ethers such as diethyl ether, methyl-tert-butyl ether, ketones such as acetone, methyl ethyl ketone, methyl isobutyl ketone, hydrocarbons such as toluene, benzene, cyclohexane or alkanes; and mixtures thereof; preferably dimethyl sulfoxide, acetone, or toluene, more preferably the solvent is dimethyl sulfoxide, followed by its addition to the reaction mixture of step (ii) at ambient temperature;
(iv) stirring the reaction mass of step (iii) for a period of about 10-60 minutes, preferably for a period of about 10-15 minutes;
(v) dissolving the chloropropyl amine of Formula VII in an aprotic solvent such as dimethyl sulfoxide (DMSO), THF (tetrahydrofuran), DMF (dimethyl formide), NMP (N-methyl pyrrolidone), ethers such as diethyl ether or methyl tert-butyl ether, ketones such as acetone, methyl ethyl ketone, or methyl isobutyl ketone, hydrocarbons such as toluene, benzene, cyclohexane or alkanes, and mixtures thereof, preferably dimethyl sulfoxide or acetone or toluene, more preferably dimethyl sulfoxide, followed by addition to the reaction mixture of step (4) at ambient temperature;
(vi) heating the reaction mass of step (v) to a temperature about 40-140°C for a period of about 1 to 6 hrs; preferably a temperature about 40-45°C for about 1 to 1.5 hrs;
(vii) quenching of the reaction mass of step (vi) using ice-cold water at a temperature about -5 to 25 °C, preferably at a temperature about 0 to 5°C;
(viii) extraction of the compound from reaction mass of step (vii) with an organic solvent such as dichloromethane, chloroform, dichloroethane, toluene, xylene, ethyl acetate, Isopropyl ether, methyl tert-butyl ether, diethyl ether or petroleum ether, preferably toluene;
(ix) distilling off the solvent under reduced pressure from the reaction solution of step (viii) to get a residue;
(x) suspending the residue of step (ix) in water, followed by adjusting pH of the solution to a value between about 2 and 6 with an acid such as hydrochloric acid, acetic acid, sulfuric acid; preferably hydrochloric acid, followed by washing with a suitable organic solvent such as dichloroethane, chloroform, dichloromethane, toluene, xylene, ethyl acetate, Isopropyl ether, methyl tert-butyl ether, diethyl ether or petroleum ether; preferably toluene;
(xi) adjusting the aqueous solution of step (x) to a pH about 8 to 13 with a solution of a base such as sodium hydroxide, sodium carbonate, sodium bicarbonate, potassium hydroxide, potassium carbonate, or potassium bicarbonate;
(xii) extracting the compound from the basified aqueous layer of step (xi) with a suitable organic solvent such as dichloromethane, chloroform, dichloroethane, toluene, xylene, ethyl acetate, isopropyl ether, methyl tert-butyl ether, diethyl ether and petroleum ether; preferably with toluene;
(xiii) distilling the solvent from the reaction solution of step (xii) to obtain a didesmethylcitalopram compound of Formula II in the form of thick syrup;
(xiv) didesmethylcitalopram is optionally purified by converting the free base into its corresponding acid addition salts in a suitable solvent such as alcohols, including methanol, ethanol, isopropanol, or butanol, or a ketone such as acetone, methyl ethyl ketone, methyl isobutyl ketone, chlorinated solvents such as dichloroethane, dichloromethane, chloroform, esters such as ethyl acetate, nitriles such as acetonitrile, or hydrocarbons such as toluene, benzene, cyclohexane, heptane, hexanes, or xylene, followed by drying to obtain the acid addition salt of compound of formula II;
(xv) hydrolyzing the dried or wet salt of formula II of the step (xiv) in water with suitable alkaline solution comprising sodium hydroxide, sodium carbonate, sodium bicarbonate, potassium hydroxide, potassium carbonate or potassium bicarbonate; preferably using sodium hydroxide solution;
(xvi) extracting the compound of formula II from the solution of step (xv) in a suitable organic solvent selected from dichloromethane, chloroform, dichloroethane, toluene, ethyl acetate, isopropyl ether, methyl tert-butyl ether, diethyl ether and petroleum ether; preferably with toluene; and
(xvii) distilling the organic solvent of step (xvi) to afford the compound of Formula II.

Alternatively, escitalopram can be prepared by: (i) dissolving racemic 1-(3-aminopropyl)-1-(4-fluorophenyl)-1,3-dihydroisobenzofuran-5-carbonitrile of Formula II in a suitable solvent such alcohols, such as methanol, ethanol, isopropanol, butanol, or ketones such as acetone, ethyl methyl ketone, methyl isobutyl ketone, chlorinated solvents such as dichloroethane, dichloromethane, chloroform, esters such as ethyl acetate, nitriles such as acetonitrile, hydrocarbons such as toluene, benzene, cyclohexane, heptane, hexanes, xylene or alkanes, and mixtures thereof; preferably acetonitrile; (ii) stirring the reaction mass of step (i) for a period about 5 to 60 minutes, preferably for a period about 10 to 15 minutes; (iii) dissolving an enantiomerically pure acid such as optical antipodes of tartaric acids such as di-benzoyltartaric acid, di-p-toluyl tartaric acid and o-nitrobenzoyl tartaric acid, lactic acid, bisnaphthylphosphoric acid, camphorsulfonic acid, such as 10-camphorsulfonic acid and 8-camphorsulfonic acid, malic acid, N-acetyl glutamic acid, mandelic acid and the like, conveniently tartaric acid antipodes; and preferably (-)-di-p-toluoyl tartaric acid, in a suitable solvent such as alcohols, such as methanol, ethanol, isopropanol, butanol, ketones such as acetone, methyl ethyl ketone, methyl isobutyl ketone, chlorinated solvents such as dichloroethane, dichloromethane, or chloroform, esters such as ethyl acetate, nitriles such as acetonitrile, hydrocarbons such as toluene, benzene, cyclohexane, heptane, hexanes, xylene or alkanes, and mixtures thereof; preferably acetonitrile, at ambient temperature; (iv) addition of the reaction mass of step (iii) to a reaction mass of step (ii) and reacting for a period about 10 to 60 minutes, preferably about 10 to 15 minutes; (v) heating the reaction mass of step (iv) to a temperature about 40-100°C for a period about 1 to 6 hours; preferably a temperature about 60-65°C for about 45 to 60 minutes; (vi) cooling the reaction mass of step (v) to a temperature about -20 to 10°C for a period of about 1 to 3 hours; preferably -5 to 5°C for 1 to 1.5 hours; (vii) filtering the obtained solid from step (vi) followed by washing with a solvent described in step (iii); (viii) drying the solid obtained in step (vii) at temperature about 40 to 70°C for a period about 2 to 48 hours; preferably about 60-65°C for about 6 to 8 hours to afford a diastereomeric salt of compound of Formula (II); (ix) dissolving the diastereomeric salt obtained in step (viii) in a suitable solvent such as water, alcohols, such as methanol, ethanol, isopropanol, butanol, ketones such as acetone, methyl ethyl ketone, or methyl isobutyl ketone, chlorinated solvents such as dichloroethane, dichloromethane, or chloroform, esters such as ethyl acetate, nitriles such as acetonitrile, hydrocarbons such as toluene, benzene, cyclohexane, heptane, hexanes, xylene or alkanes, and mixture thereof; preferably in a mixture of 15:1 acetonitrile and water; (x) heating the reaction mass of step (ix) to a temperature about 40-100°C for a period about 1 to 6 hours, preferably a temperature of 60-65°C for 45 to 60 minutes; (xi) cooling the reaction mass of step (x) to a temperature about -20 to +30°C for a period about 1 to 72 hours, preferably -5 to 5°C for 1 to 3 hours; (xii) filtering the obtained solid from step (xi) followed by washing with a solvent described in step (ix); (xiii) drying the solid obtained in step (xii) at temperature about 40 to 70°C for a period about 2 to 48 hours, preferably about 60-65°C for about 6 to 8 hours, to afford (+)-1-(3-aminopropyl)-1-(4-fluorophenyl)-1,3-dihydroisobenzofuran-5-carbonitrile in the form of a (-)-DPTTA salt of compound of Formula (XI); (xiv) suspending the solid obtained in step (xiii) in water followed by adjusting pH of the solution between about 8 to 13 with a base such as sodium hydroxide, sodium carbonate, sodium bicarbonate, potassium hydroxide, potassium carbonate or potassium bicarbonate, preferably using sodium hydroxide solution; (xv) extracting the compound of formula XI from the basic aqueous layer of step (xv) with a suitable organic solvent such as dichloromethane, chloroform, dichloroethane, toluene, xylene, ethyl acetate, isopropyl ether, methyl tert-butyl ether, diethyl ether or petroleum ether, preferably with toluene; (xvi) distilling the solvent from the reaction solution of step (xv) to obtain (+)-didesmethylcitalopram of Formula XI; (xvii) reacting the compound formula XI in step (xvi) with a suitable ethylating agent such as methyl iodide, dimethyl sulfate, or a mixture of formic acid and formaldehyde, at a temperature about 20 to 150 °C for about 4 to 24 hours, preferably with formic acid and formaldehyde at about 80 to 100°C for about 10 to 18 hours, more preferably with formic acid and formaldehyde at about 95 to 100°C for about 12 hours; (xviii) cooling the reaction mass of step (xvii) to ambient temperature followed by addition of an inorganic acid such as hydrochloric acid or sulfuric acid; (xix) distilling the reaction solution of step (xviii) to obtain a thick residue; (xx) suspending the residue of step (xix) in a basic solution comprising sodium hydroxide, sodium carbonate, sodium bicarbonate, potassium hydroxide, potassium carbonate or potassium bicarbonate, preferably aqueous sodium hydroxide; (xxi) extracting the compound from the basic aqueous layer of step (xx) with an organic solvent such as dichloromethane, chloroform, dichloroethane, toluene, ethyl acetate, isopropyl ether, methyl tert-butyl ether, diethyl ether, or petroleum ether; preferably diethyl ether; and (xxii) distilling the solvent from the reaction solution of step (xxi) to afford (+)-1-[3-(N,N'-dimethylamino)propyl]-1-(4-fluorophenyl)-1,3-dihydro-5-isobenzofurancarbonitrile of Formula I.

R-citalopram of formula V can be prepared by;
(i) dissolving racemic 1-(3-aminopropyl)-1-(4-fluorophenyl)-1,3-dihydroisobenzofuran-5-carbonitrile of Formula II in a suitable solvent such as alcohols, such as methanol, ethanol, isopropanol, butanol or ketones such as acetone, methyl ethyl ketone, methyl isobutyl ketone, chlorinated solvents such as dichloroethane, dichloromethane, chloroform, esters such as ethyl acetate, nitriles such as acetonitrile, hydrocarbons such as toluene, benzene, cyclohexane, heptane, hexanes, xylene or alkanes, and mixture thereof; preferably acetonitrile;
(ii) stirring the solution of step (i) for a period of about 5 to 60 minutes, preferably for a period of about 10 to 15 minutes;
(iii) dissolving an enantiomerically pure acid, such as optical antipodes of tartaric acids such as di-benzoyltartaric acid, di-p-toluoyl tartaric acid and o-nitrobenzoyl tartaric acid, lactic acid, bisnaphthylphosphoric acid, camphorsulfonic acid, such as 10-camphorsulfonic acid and 8-camphorsulfonic acid, malic acid, N-acetyl glutamic acid, mandelic acid and the like, preferably tartaric acid antipodes; more preferably (+)-di-p-toluoyl tartaric acid, in a suitable solvent such as alcohols, such as methanol, ethanol, isopropanol, or butanol, or ketones such as acetone, methylethyl ketone, methyl isobutyl ketone, chlorinated solvents such as dichloroethane, dichloromethane, chloroform, esters such as ethyl acetate, nitriles such as acetonitrile, hydrocarbons such as toluene, benzene, cyclohexane, heptane, hexanes, xylene or alkanes, and mixture thereof; preferably acetonitrile at ambient temperatures;
(iv) adding the solution of step (iii) to the solution of step (ii) for a period of about 10 to 60 minutes, preferably about 10 to 15 minutes;
(v) heating the reaction mixture of step (iv) to a temperature about 40-100°C for a period of about 1 to 6 hours; preferably a temperature about 60-65°C for about 45 to 60 minutes;
(vi) cooling the reaction mass of step (v) to a temperature about -20 to 40°C for a period of about 1 to 3 hours; preferably about -5 to 5°C for about 1 to 1.5 hours;
(vii) filtering to recover the obtained solid from step (vi), followed by its washing with a solvent as specified in step (ii);
(viii) drying the solid obtained in step (vii) at temperature about 40 to 70°C for a period of about 2 to 48 hours; preferably about 60-65°C for about 6 to 8 hours to afford the diastereomeric salt of the compound of Formula II;
(ix) dissolving the diastereomeric salt obtained in step (viii) in a suitable solvent such as water, alcohols, such as methanol, ethanol, isopropanol, or butanol, or ketones such as acetone, methyl ethyl ketone, methyl isobutyl ketone, chlorinated solvents such as dichloroethane, dichloromethane, or chloroform, esters such as ethyl acetate, nitriles such as acetonitrile, hydrocarbons such as toluene, benzene, cyclohexane, heptane, hexanes, xylene or alkanes, and mixtures thereof; preferably an acetonitrile and water combination at ambient temperatures;
(x) heating the solution of step (ix) to a temperature about 40-100°C for a period of about 1 to 6 hours; preferably about 60-65°C for about 45 to 60 minutes;
(xi) cooling the solution of step (x) a temperature about -20 to 10°C for a period of about 1 to 8 hours; preferably about -5 to 5°C for about 1 to 3 hours;
(xii) filtering the obtained solid in step (xi) followed by its washing with a solvent as specified in step (ix);
(xiii) drying the solid obtained in step (xii) at temperature about 40 to 70°C for a period of about 2 to 48 hours; preferably about 60-65°C for about 6 to 8 hours to afford a solid of (-)-1-(3-aminopropyl)-1-(4-fluorophenyl)-1,3-dihydroisobenzofuran-5-carbonitrile in the form of a (+)-DPTTA salt of the compound of Formula XII;
(xiv) suspending the solid obtained in step (xiii) in water followed by adjusting pH of the solution to a value about 8 to 13 with a solution comprising sodium hydroxide, sodium carbonate, sodium bicarbonate, potassium hydroxide, potassium carbonate or potassium bicarbonate; preferably using sodium hydroxide solution;
(xv) extracting the compound from the basic aqueous layer of step (xiv) with a suitable organic solvent such as dichloromethane, chloroform, dichloroethane, toluene, xylene, ethyl acetate, isopropyl ether, methyl tert-butyl ether, diethyl ether or petroleum ether; preferably with toluene;
(xvi) distilling the solvent from the solution of step (xv) to obtain (-)-didesmethylcitalopram of compound of Formula XII;
(xvii) reacting the compound having formula (XII) from step (xvi) with suitable methylating agents such as methyl iodide, dimethyl sulfate or a mixture of formic acid and formaldehyde at a temperature about 20 to 150 °C for about 4 to 24 hours, preferably with formic acid and formaldehyde about 80 to 100°C for about 10 to 18 hours, more preferably with formic acid and formaldehyde about 95 to 100°C for about 12 hours;
(xviii) cooling the reaction mass of step (xvii) to ambient temperature followed by addition of an inorganic acid such as hydrochloric acid or sulfuric acid, preferably hydrochloric acid;
(xix) distilling the acidified mixture of step (xviii) to obtain a thick residue;
(xx) suspending the residue of step (xix) in a basic solution comprising sodium hydroxide, sodium carbonate, sodium bicarbonate, potassium hydroxide, potassium carbonate or potassium bicarbonate, preferably aqueous sodium hydroxide;
(xxi) extracting the compound from the basic aqueous layer of step (xx) with an organic solvent such as dichloromethane, chloroform, dichloroethane, toluene, ethyl acetate, isopropyl ether, methyl tert-butyl ether, diethyl ether or petroleum ether; preferably diethyl ether; and
(xxii) distilling the solvent from the reaction solution of step (xxi) to afford (-)-1-[3-(N,N'-dimethylamino) propyl]-1-(4-fluorophenyl)-1,3-dihydro-5-isobenzofurancarbonitrile of formula V.

Citalopram of formula VIII can be prepared by:
(i) heating a solution of a base such as LDA (lithium diisopropyl amine), NaH, n-BuLi, metal oxides such as NaOMe, KOMe, LiOMe, NaO-*t*-Bu, KO-*t*-Bu and LiO-*t*-Bu preferably NaH, or KO-*t*-Bu or LDA, more preferably in KO-*t*-Bu in an aprotic solvent such as DMSO, THF (tetrahydrofuran), DMF (dimethyl formide), NMP (N-methyl pyrrolidone), ethers such as diethyl ether or methyl tert-butyl ether, ketones such as acetone, methyl ethyl ketone, or methyl isobutyl ketone, hydrocarbons such as toluene, benzene, cyclohexane or alkanes, and mixtures thereof; preferably dimethyl sulfoxide or acetone or toluene, more preferably the solvent is dimethyl sulfoxide, in a anhydrous condition under nitrogen atmosphere, at a temperature about 50-120°C; preferably about 60-65°C for more than an hour;
(ii) cooling the reaction mixture of step (i) to temperature about 10-50°C, preferably to a temperature about 30-35°C;
(iii) dissolving 5-cyano-1-(4-fluoro phenyl)-1,3-dihydrobenzofuran of formula V in a suitable aprotic solvent such as dimethyl sulfoxide (DMSO), THF (tetrahydrofuran), DMF (dimethyl formide), NMP (N-methyl pyrrolidone), ethers such as diethyl ether, methyl tert-butyl ether, ketones such as acetone, methyl ethyl ketone, or methyl isobutyl ketone, hydrocarbons such as toluene, benzene, cyclohexane or alkanes, and mixtures thereof; preferably dimethyl sulfoxide or acetone or toluene, more preferably the solvent is dimethyl sulfoxide, followed by addition of the solution to the reaction mixture of step (ii) at ambient temperature;
(iv) stirring the reaction mass of step (iii) for a period of about 10-60 minutes, preferably for a period about 10-15 minutes;
(v) dissolving the chloropropyl amine of formula VI in an aprotic solvent such as dimethyl sulfoxide (DMSO), THF (tetrahydrofuran), DMF (dimethyl formide), NMP (N-methyl pyrrolidone), ethers such as diethyl ether, methyl tert-butyl ether, ketones such as acetone, methyl ethyl ketone, or methyl isobutyl ketone, hydrocarbons such as toluene, benzene, cyclohexane or alkanes, and mixtures thereof; preferably dimethyl sulfoxide or acetone or toluene, more preferably the solvent is dimethyl sulfoxide, followed by addition of the solution to the reaction mixture of step (iv) at ambient temperature;
(vi) heating the reaction mass of step (v) to a temperature about 40-140°C for a period of about 1 to 6 hrs; preferably a temperature about 40-45°C for about 1 to 1.5 hrs;
(vii) quenching the reaction mass of step (vi) using ice cold water at a temperature about -5 to 25 °C, preferably at a temperature about 0 to 5°C;
(viii) extraction of the compound from the reaction mass of step (vii) with an organic solvent such dichloromethane, chloroform, dichloroethane, toluene, xylene, ethyl acetate, isopropyl ether, methyl tert-butyl ether, diethyl ether or petroleum ether, preferably toluene;
(ix) distilling off the solvent under vacuum from the reaction solution of step (viii) to obtain a residue;
(x) suspending the residue of step (ix) in water followed by adjusting pH of the solution to a value between about 2 and 6 with an acid such as hydrochloric acid, acetic acid, or sulfuric acid; preferably hydrochloric acid solution, followed by washing the acid solution with a suitable organic solvent such as dichloroethane, chloroform, dichloromethane, toluene, xylene, ethyl acetate, Isopropyl ether, methyl tert-butyl ether, diethyl ether or petroleum ether; preferably toluene;
(xi) adjusting the aqueous solution of step (x) to a pH about 8-13 with a base such as sodium hydroxide, sodium carbonate sodium bicarbonate, potassium hydroxide, potassium carbonate or potassium bicarbonate; preferably using sodium hydroxide solution;
(xii) extracting the compound from the basic aqueous layer of step (xi) with a suitable organic solvent such as dichloromethane, chloroform, dichloroethane, toluene, xylene, ethyl acetate, isopropyl ether, methyl tert-butyl ether, diethyl ether or petroleum ether; preferably with toluene;
(xiii) distilling the solvent from the reaction solution of step (xii) to obtain a didesmethylcitalopram of compound of Formula II in the form of thick syrup;
(xiv) optionally purifying the Formula II compound by converting the free base into its corresponding acid addition salts in a suitable solvent such as alcohols, such as methanol, ethanol, isopropanol, or butanol, ketones such as acetone, methyl ethyl ketone, or methyl isobutyl ketone, chlorinated solvents such as dichloroethane, dichloromethane, or chloroform, esters such as ethyl acetate, nitriles such as acetonitrile, hydrocarbons such as toluene, benzene, cyclohexane, heptane, hexanes, or xylene, followed by drying to get the acid addition salt of compound of formula II;
(xv) hydrolysis of the dried or wet salt of formula II of step (xiv) in water with a suitable alkaline solution containing sodium hydroxide, sodium carbonate, sodium bicarbonate, potassium hydroxide, potassium carbonate or potassium bicarbonate; preferably using sodium hydroxide solution;
(xvi) extracting the compound of formula II from the solution of step (xv) in a suitable organic solvent such as dichloromethane, chloroform, dichloroethane, toluene, ethyl acetate, isopropyl ether, methyl tert-butyl ether, diethyl ether or petroleum ether; preferably with toluene;
(xvii) distilling the organic solvent of step (xvi) to afford the compound of formula II;
(xviii) reacting the compound formula II in step (xvii) with a suitable methylating agent such as methyl iodide, dimethyl sulphate or a mixture of formic acid and formaldehyde, at a temperature about 20 to 150 °C for about 4 to 24 hours, preferably with formic acid and formaldehyde at about 80 to 100°C for about 10 to 18 hours, more preferably with formic acid and formaldehyde about 95 to 100°C for about 12 hours;
(xix) cooling the reaction mass of step (xviii) to ambient temperature followed by addition of an inorganic acid such as hydrochloride acid or sulfuric acid, preferably hydrochloric acid;
(xx) distilling the reaction solution of step (xix) to obtain a thick residue;
(xxi) suspending the residue of step (xx) in a basic solution containing sodium hydroxide, sodium carbonate, sodium bicarbonate, potassium hydroxide, potassium carbonate or potassium bicarbonate, preferably aqueous sodium hydroxide;
(xxii) extracting the compound from the basic aqueous layer of step (xxi) with an organic solvent such as dichloromethane, chloroform, dichloroethane, toluene, ethyl acetate, isopropyl ether, methyl tert-butyl ether, diethyl ether or petroleum ether; preferably diethyl ether; and
(xxiii) distilling the solvent from the reaction solution of step (xxii) to afford racemic 1-[3-(N,N'-dimethylamino)propyl]-1-(4-fluorophenyl)-1,3-dihydro-5-isobenzofurancarbonitrile of Formula VIII.

Racemic desmethylcitalopram of Formula XIII can be prepared by:
(i) dissolving the didesmethylcitalopram of Formula II in a solvent such as an ether such as diethyl ether, methyl tert-butyl ether, a hydrocarbon such as toluene, benzene, cyclohexane or an alkane, or mixtures thereof; preferably toluene;
(ii) adding benzaldehyde to the solution of step (i) at ambient temperature;
(iii) refluxing the reaction mass of step (ii) with a water separator for a period of about 2 to 10 hours at a temperature about 100-110°C, preferably until all of the water is collected;
(iv) reacting the reaction mass of step (iii) with a methylating agent such as methyl iodide, dimethyl sulfate or a mixture of formic acid and formaldehyde, at a temperature about 20 to 150 °C for about 1 to 10 hours, preferably with dimethyl sulfate about 100 to 110°C for about 1-1.5 hours;
(v) cooling the reaction mass of step (iv) to a temperature about 90-95°C;
(vi) treating the reaction mass of step (v) with water at a temperature about 90-95°C;
(vii) refluxing the reaction mass of step (vi) for a period about 20-120 minutes at about 90-125°C, preferably about 100-110°C for about 30-40 minutes;
(viii) cooling the reaction mass of step (vii) to ambient temperature and washing with a suitable organic solvent such as dichloroethane, chloroform, dichloromethane, toluene, xylene, ethyl acetate, isopropyl ether, methyl tert-butyl ether, diethyl ether or petroleum ether; preferably diethyl ether;
(ix) adjusting the aqueous solution of step (viii) to a pH about 8-13 with a solution comprising sodium hydroxide, sodium carbonate, sodium bicarbonate, potassium hydroxide, potassium carbonate or potassium bicarbonate; preferably using sodium hydroxide solution, more preferably to a pH about 12-13;
(x) extracting the compound from the basic aqueous layer of step (ix) with a suitable organic solvent such as dichloromethane, chloroform, dichloroethane, toluene, xylene, ethyl acetate, isopropyl ether, methyl tert-butyl ether, diethyl ether or petroleum ether; preferably with diethyl ether;
(xi) wash ing the organic layer of step (x) with water followed by drying with a suitable dehydrating agents such as anhydrous sodium sulfate;
(xii) distilling the solvent from the reaction solution of step (xi) to obtain a racemic desmethylcitalopram of compound of Formula XIII in the form of thick syrup;
(xiii) optionally purifying by converting the free base into its corresponding acid addition salts in a suitable solvent such as an alcohol such as methanol, ethanol, isopropanol, or butanol, a ketone such as acetone, methyl ethyl ketone, or methyl isobutyl ketone, a chlorinated solvent such as dichloroethane, dichloromethane, or chloroform, an ester such as ethyl acetate, a nitrile such as acetonitrile, a hydrocarbon such as toluene, benzene, cyclohexane, heptane, hexanes, or xylene, followed by drying to get the acid addition salt of compound of Formula XIII.

(+)-Desmethylcitalopram of Formula IX can be prepared by:
(i) dissolving racemic 1-(3-aminopropyl)-1-(4-fluorophenyl)-1,3-dihydroisobenzofuran-5-carbonitrile of Formula II in suitable solvents such as alcohols, such as methanol, ethanol, isopropanol, or butanol, ketones such as acetone, methyl ethyl ketone, or methyl isobutyl ketone, chlorinated solvents such as dichloroethane, dichloromethane, or chloroform, esters such as ethyl acetate, nitriles such as acetonitrile, hydrocarbons such as toluene, benzene, cyclohexane, heptane, hexanes, xylene or alkanes, and mixtures thereof, preferably acetonitrile;
(ii) stirring the reaction mass of step (i) for a period of about 5 to 60 minutes, preferably for a period about 10 to 15 minutes;
(iii) dissolving an enantiomerically pure acid such as optical antipodes of tartaric acids such as di-benzoyltartaric acid, di-p-toluoyl tartaric acid and o-nitrobenzoyl tartaric acid, lactic acid, bisnaphthylphosphoric acid, camphorsulfonic acid, such as 10-camphorsulfonic acid and 8-camphorsulfonic acid, malic acid, N-acetyl glutamic acid, mandelic acid and the like, preferably tartaric acid antipodes, more preferably (-)-di-p-toluoyl tartaric acid, in a suitable solvent such as alcohols, such as methanol, ethanol, isopropanol, or butanol, ketones such as acetone, methyl ethyl ketone, or methyl isobutyl ketone, chlorinated solvents such as dichloroethane, dichloromethane, or chloroform, esters such as ethyl acetate, nitriles such as acetonitrile, hydrocarbons such as toluene, benzene, cyclohexane, heptane, hexanes, xylene or alkanes, and mixtures thereof; preferably acetonitrile, at ambient temperatures;
(iv) adding the reaction mass of step (iii) to the reaction mass of step (ii) for a period of about 10 to 60 minutes, preferably about 10 to 15 minutes;
(v) heating the reaction mass of step (iv) to a temperature about 40-100°C for a period of about 1 to 6 hours; preferably a temperature about 60-65°C for about 45 to 60 minutes;
(vi) cooling the reaction mass of step (v) a temperature about -20 to 10°C for a period of about 1 to 3 hours; preferably about -5 to 5°C for about 1 to 1.5 hours;
(vii) filtering the obtained solid in step (vi) followed by washing with a solvent such as is used in step (i);
(viii) drying the solid obtained in step (vii) at temperature about 40 to 70°C for a period of about 2 to 48 hours; preferably about 60 -65°C for about 6 to 8 hours to afford a solid of the diastereomeric salt of the compound of Formula II;
(ix) dissolving the diastereomeric salt obtained in step (viii) in a suitable solvent such as water, alcohols such as methanol, ethanol, isopropanol, or butanol, ketones such as acetone, methyl ethyl ketone, or methyl isobutyl ketone, chlorinated solvents such as dichloroethane, dichloromethane, or chloroform, esters such as ethyl acetate, nitriles such as acetonitrile, hydrocarbons such as toluene, benzene, cyclohexane, heptane, hexanes, xylene or alkanes, and mixture thereof, preferably an acetonitrile and water combination at ambient temperatures;
(x) heating the reaction mass of step (ix) to a temperature about 40 to 100°C for a period of about 1 to 6 hours; preferably a temperature about 60 to 65°C for about 45 to 60 minutes;
(xi) cooling the reaction mass of step (x) a temperature about -20 to 10°C for a period of about 1 to 8 hours; preferably about -5 to 5°C for about 1 to 3 hours;
(xii) filtering the obtained solid in step (xi) followed by washing with a solvent such as is used in step (ix);
(xiii) drying the solid obtained in step (xii) at temperature about 40 to 70°C for a period of about 2 to 48 hours; preferably about 60-65°C for about 6 to 8 hours, to afford a solid of (+)-1-(3-aminopropyl)-1-(4-fluorophenyl)-1,3-dihydroisobenzofuran-5-carbonitrile in the form of a DPTTA salt of the compound of Formula (XI);
(xiv) dissolving the solid obtained in step (xiii) in water, followed by adjusting pH of the solution to a value between about 8 and 13 with a base such as sodium hydroxide, sodium carbonate, sodium bicarbonate, potassium hydroxide, potassium carbonate or potassium bicarbonate; preferably using sodium hydroxide solution;
(xv) extracting the compound from the basic aqueous layer of step (xiv) with a suitable organic solvent such as dichloromethane, chloroform, dichloroethane, toluene, xylene, ethyl acetate, isopropyl ether, methyl tert-butyl ether, diethyl ether and petroleum ether; preferably with toluene;
(xvi) distilling the solvent from the reaction solution of step (xv) to obtain the (+)-didesmethylcitalopram compound of Formula (XI);
(xvii) dissolving the (+)-didesmethylcitalopram of Formula XI in a solvent such as ethers such as diethyl ether or methyl tert-butyl ether, hydrocarbons such as toluene, benzene, cyclohexane or alkanes, and mixtures thereof; preferably toluene;
(xviii) adding benzaldehyde to the reaction mass of step (xvii) at ambient temperatures;
(xix) refluxing the above reaction mass of step (xviii) with a water separator for a period of about 2 to 10 hours at a temperature about 100-110°C, preferably until all of the water is collected;
(xx) reacting the reaction mass of step (xix) with a suitable methylating agent such as methyl iodide, dimethyl sulfate, or a mixture of formic acid and formaldehyde, at a temperature about 20 to 150 °C for about 1 to 10 hours, preferably with dimethyl sulfate at about 100 to 110°C for about 1 to 1.5 hours;
(xxi) cooling the reaction mass of step (xx) to a temperature about 90-95°C;
(xxii) treating the reaction mass of step (xxi) with water at a temperature about 90-95°C;
(xxiii) refluxing the reaction mass of step (xxii) for a period of about 20-120 minutes at about 90-125°C, preferably 100-110°C for 30-40 minutes;
(xxiv) cooling the reaction mass of step (xxiii) to ambient temperature and washing with a suitable organic solvent such as dichloroethane, chloroform, dichloromethane, toluene, xylene, ethyl acetate, Isopropyl ether, methyl tert-butyl ether, diethyl ether or petroleum ether; preferably diethyl ether;
(xxv) adjusting the aqueous solution of step (xxiv) to a pH about 8-13 with a base such as sodium hydroxide, sodium carbonate, sodium bicarbonate, potassium hydroxide, potassium carbonate or potassium bicarbonate; preferably using sodium hydroxide solution, more preferably to a pH about 12-13;
(xxvi) extracting the compound from the basic aqueous layer of step (xxv) with a suitable organic solvent such as dichloromethane, chloroform, dichloroethane, toluene, xylene, ethyl acetate, isopropyl ether, methyl tert-butyl ether, diethyl ether or petroleum ether; preferably with diethyl ether;
(xxvii) washing the organic layer of step (xxvi) with water followed by drying with suitable dehydrating agents such as anhydrous sodium sulfate;
(xxviii) distilling the solvent from the reaction solution of step (xxvii) to obtain (+)-desmethylcitalopram of Formula IX in the form of thick syrup; and
(xxix) optionally purifying by converting the free base into its corresponding acid addition salt in a suitable solvent such as alcohols such as methanol, ethanol, isopropanol, or butanol, ketones such as acetone, methyl ethyl ketone, or methyl isobutyl ketone, chlorinated solvents such as dichloroethane, dichloromethane, or chloroform, esters such as ethyl acetate, nitriles such as acetonitrile, hydrocarbons such as toluene, benzene, cyclohexane, heptane, hexanes, or xylene, followed by drying to get the acid addition salt of compound of formula IX.

(-)-Desmethylcitalopram of formula X can be prepared by:
(i) dissolving racemic 1-(3-aminopropyl)-1-(4-fluorophenyl)-1,3-dihydroisobenzofuran-5-carbonitrile of Formula II in a suitable solvent such as alcohols such as methanol, ethanol, isopropanol, or butanol, ketones such as acetone, methyl ethyl ketone, or methyl isobutyl ketone, chlorinated solvents such as dichloroethane, dichloromethane, or chloroform, esters such as ethyl acetate, nitriles such as acetonitrile, hydrocarbons such as toluene, benzene, cyclohexane, heptane, hexanes, xylene or alkanes, and mixture thereof; preferably acetonitrile;
(ii) stirring the reaction mass of step (i) for a period of about 5 to 60 minutes, preferably for a period of about 10 to 15 minutes;
(iii) dissolving an enantiomerically pure acid such as optical antipodes of tartaric acids such as di-benzoyl tartaric acid, di-p-toluoyl tartaric acid or o-nitrobenzoyl tartaric acid, lactic acid, bisnaphthylphosphoric acid, camphorsulfonic acids, such as 10-camphorsulfonic acid and 8-camphorsulfonic acid, malic acid, N-acetyl glutamic acid, mandelic acid and the like, preferably tartaric acid antipodes; more preferably (+)-di-p-toluoyl tartaric acid, in a suitable solvent such as alcohols such as methanol, ethanol, isopropanol, or butanol, ketones such as acetone, methyl ethyl ketone, or methyl isobutyl ketone, chlorinated solvents such as dichloroethane, dichloromethane, or chloroform, esters such as ethyl acetate, nitriles such as acetonitrile, hydrocarbons such as toluene, benzene, cyclohexane, heptane, hexanes, xylene or alkanes, and mixtures thereof; preferably acetonitrile at a ambient temperatures;
(iv) adding the reaction mass of step (iii) to the reaction mass of step (ii) for a period of about 10 to 60 minutes, preferably about 10 to 15 minutes;
(v) heating the reaction mass of step (iv) to a temperature about 40-100°C for a period of about 1 to 6 hours; preferably a temperature about 60 -65°C for about 45 to 60 minutes;
(vi) cooling the reaction mass of step (v) to a temperature about -20 to 10°C for a period of about 1 to 3 hours; preferably about -5 to 5°C for about 1 to 1.5 hours;
(vii) filtering the obtained solid in step (vi) followed by washing with a solvent such as is used in step (i);
(viii) drying the solid obtained in step (vii) at temperature about 40 to 70°C for a period of about 2 to 48 hours; preferably about 60 to 65°C for about 6 to 8 hours, to afford a diastereomeric salt of the compound of Formula II;
(ix) dissolving the diastereomeric salt obtained in step (viii) in a suitable solvent such as water, alcohols such as methanol, ethanol, isopropanol, or butanol, ketones such as acetone, methyl ethyl ketone, or methyl isobutyl ketone, chlorinated solvents such as dichloroethane, dichloromethane, or chloroform, esters such as ethyl acetate, nitriles such as acetonitrile, hydrocarbons such as toluene, benzene, cyclohexane, heptane, hexanes, xylene or alkanes, and mixture thereof, preferably an acetonitrile and water combination at ambient temperatures;
(x) heating the reaction mass of step (ix) to a temperature about 40-100°C for a period of about 1 to 6 hours; preferably a temperature about 60-65°C for about 45 to 60 minutes;
(xi) cooling the reaction mass of step (x) a temperature about -20 to 10°C for a period of about 1 to 8 hours, preferably about -5 to 5°C for about 1 to 3 hours;
(xii) filtering the obtained solid in step (xi) followed by washing with a solvent such as is used in step (ix);
(xiii) drying the solid obtained in step (xii) at temperature about 40 to 70°C for a period of about 2 to 48 hours, preferably about 60-65°C for about 6 to 8 hours, to afford a solid of (-)-1-(3-aminopropyl)-1-(4-fluorophenyl)-1,3-dihydroisobenzofuran-5-carbonitrile in the form of a DPTTA salt;
(xiv) dissolving the solid obtained in step (xiii) in water, followed by adjusting pH of the solution to a value between about 8 and 13 with a base such as sodium hydroxide, sodium carbonate, sodium bicarbonate, potassium hydroxide, potassium carbonate or potassium bicarbonate; preferably using sodium hydroxide solution, preferably to pH values about 12-13;
(xv) extracting the compound from the basic aqueous layer of step (xiv) with an organic solvent such as dichloromethane, chloroform, dichloroethane, toluene, xylene, ethyl acetate, isopropyl ether, methyl tert-butyl ether, diethyl ether, and petroleum ether, preferably with toluene;
(xvi) distilling the solvent from the reaction solution of step (xv) to obtain the compound (-)-didesmethylcitalopram of Formula XII;
(xvii) dissolving the (-)-didesmethyl citalopram of Formula XII in a solvent such as ethers such as diethyl ether or methyl tert-butyl ether, hydrocarbons such as toluene, benzene, cyclohexane or alkanes, and mixtures thereof; preferably toluene;
(xviii) adding benzaldehyde to the reaction mass of step (xvii) at ambient temperature;
(xix) refluxing the above reaction mass of step (xviii) with a water separator for a period of about 2 to 10 hours at a temperature about 100-110°C, preferably until all of the water is collected;
(xx) reacting the reaction mass of step (xix) with a suitable methylating agent such as methyl iodide, dimethyl sulfate or a mixture of formic acid and formaldehyde, at a temperature about 20 to 150 °C for about 1 to 10 hours, preferably with dimethyl sulfate at about 100 to 110°C for about 1-1.5 hours;
(xxi) cooling the reaction mass of step (xx) to a temperature about 90-95°C;
(xxii) treating the reaction mass of step (xxi) with water at a temperature about 90-95°C;
(xxiii) refluxing the reaction mass of step (xxii) for a period of about 20 to 120 minutes at about 90 to 125°C, preferably about 100-110°C for about 30-40 minutes;
(xxiv) cooling the reaction mass of step (xxiii) to ambient temperature and washing with a suitable organic solvent such as dichloroethane, chloroform, dichloromethane, toluene, xylene, ethyl acetate, Isopropyl ether, methyl tert-butyl ether, diethyl ether or petroleum ether; preferably diethyl ether;
(xxv) adjusting the aqueous solution of step (xxiv) to a pH about 8-13 with a base such as sodium hydroxide, sodium carbonate, sodium bicarbonate, potassium hydroxide, potassium carbonate, or potassium bicarbonate, preferably using sodium hydroxide solution, more preferably to a pH about 12-13;
(xxvi) extracting the compound from the basic aqueous layer of step (xxv) with a suitable organic solvent such as dichloromethane, chloroform, dichloroethane, toluene, xylene, ethyl acetate, isopropyl ether, methyl tert-butyl ether, diethyl ether and petroleum ether, preferably with diethyl ether;
(xxvii) washing the organic layer of step (xxvi) with water followed by drying with a suitable dehydrating agents such as anhydrous sodium sulfate;
(xxviii) distilling the solvent from the reaction solution of step (xxvii) to obtain (-)-desmethylcitalopram of Formula X in the form of thick syrup; and
(xxix) optionally purifying by converting the free base into its corresponding acid addition salt in a suitable solvent such as alcohols such as methanol, ethanol, isopropanol, or butanol, ketones such as acetone, methyl ethyl ketone, or methyl isobutyl ketone, chlorinated solvents such as dichloroethane, dichloromethane, or chloroform, esters such as ethyl acetate, nitriles such as acetonitrile, hydrocarbons such as toluene, benzene, cyclohexane, heptane, hexanes, or xylene, followed by drying to get the acid addition salt compound of formula X.

In the foregoing discussion and in the following examples, various processing and reaction conditions such as temperatures and times are given to exemplify useful conditions. Those skilled in the art will recognize that significant deviations can be made in many instances, without altering the final results. Also, there are suitable known alternatives to certain of the process steps, such as substituting centrifugation for filtration; such will be readily apparent to those skilled in the art and are included within the scope of the invention.

The following examples are illustrative of the above-described processes and are not intended to limit the scope of the invention as defined by the appended claims.

### EXAMPLE 1

The compound 1-(3-aminopropyl)-1-(4-fluorophenyl)-1,3-dihydroisobenzofuran-5-carbonitrile (Formula II) was prepared, as follows:
A solution was prepared by adding 7.5 g of potassium tertiary butoxide in DMSO (40 ml) at 60-65°C under nitrogen atmosphere. To the resulting solution a solution of 1-(4-fluorophenyl)-1,3-dihydroisobenzofuran-5-carbonitrile (10 g) in DMSO (35 ml) was added within 10 minutes at 25 -30°C. After maintaining for 15-20 minutes, a solution of 3-chloropropyl amine (12 g) in DMSO (2.5 ml) was added at once at a temperature between 25-30°C. After the addition is over, the reaction mixture was heated slowly to 40-45°C for 60 to 70 minutes. The reaction mixture was then quenched with ice-cold water (200 ml) and extracted with toluene (100 ml). The aqueous layer was again extracted with toluene (3 x 100 ml). The toluene layer was dried over anhydrous sodium sulfate and distilled off under vacuum below 65°C to obtain a thick syrup. The resulting residue was suspended in 50 ml water and acidified to a pH 2-3 with 10% aqueous hydrochloric acid solution. The resulting acidic solution was then washed with toluene (4 x 100 ml). The aqueous layer was adjusted with 10% aqueous sodium hydroxide solution to pH 10-11 and extracted with toluene (3 x 100 ml). The combined toluene layer was washed with water (2 x 100 ml), followed by distillation of the toluene layer to afford the compound of the formula II as wine red syrup (10 g).

### EXAMPLE 2

The compound 1-(3-aminopropyl)-1-(4-fluorophenyl)-1,3-dihydroisobenzofuran-5-carbonitrile (Formula II) was prepared, as follows:
A solution was prepared by adding 7.5 grams of potassium tertiary butoxide to acetone (40 ml) at 60-65°C under a nitrogen atmosphere. To the resulting solution, a solution of 1-(4-fluorophenyl)-1,3-dihydroiso benzofuran-5-carbonitrile (10 g) in acetone (35 ml) was added within 10 minutes, at 25 -30 °C. After maintaining for 15-20 minutes, a solution of 3-chloropropyl amine (12 g) in acetone (2.5 ml) was added at once, at a temperature between 25-30°C. After the addition was completed, the reaction mixture was heated slowly to 40-45°C for 60 to 70 minutes. The reaction mixture was then quenched with ice-cold water (200 ml) and extracted with toluene (100 ml). The aqueous layer was again extracted with toluene (3 x 100 ml). The toluene layer was dried over anhydrous sodium sulfate and distilled off under vacuum below 65°C to obtain a thick syrup. Then the syrup was suspended in 50 ml water and acidified to a pH 2-3 with 10% aqueous hydrochloric acid solution. The resulting acidic solution was then washed with toluene (4 x 100 ml). The aqueous layer was adjusted with 10% aqueous sodium hydroxide solution to pH 10-11 and extracted with toluene (3 x 100 ml). The combined toluene layer was washed with water (2 x 100 ml), followed by distillation of the toluene layer to afford the compound of formula II, as a wine red syrup (8 g).

### EXAMPLE 3

The compound (+)-1-(3-aminopropyl)-1-(4-fluorophenyl)-1,3-dihydroisobenzofuran-5-carbonitrile (Formula XI) was prepared, as follows:
1-((3-aminopropyl)-1-(4-fluorophenyl)-1,3-dihydroisobenzofuran-5-carbonitrile (10 g) was dissolved in 50 ml of acetonitrile. A solution of (-)-di-p-toluoyltartaric acid (13 g in 20 ml of acetonitrile) was added slowly at ambient temperature. The reaction mixture was stirred at ambient temperature to obtain a thick solid. The reaction mixture was heated to 60 -65°C for 45 to 60 minutes and the resulting the reaction mass was cooled to 0-5°C. The reaction mass was stirred at 0-5°C for 1 hour and the resulting solid was filtered under vacuum. The wet solid was then dried at 60-65°C for 6 to 8 hours, suspended in 150 ml of acetonitrile and heated to reflux. Water (10 ml) was added under reflux to obtain a clear solution. The reaction mixture was cooled to 0-5°C for 3 hours. The precipitated solid was filtered off and suspended in 100 ml of water, the pH of the suspension was adjusted to 12, and then it was extracted with toluene (3 x 100 ml). Combined toluene layers were washed with water (2 x 50 ml), dried over anhydrous sodium sulfate, and distilled to afford (+)-1-(3-aminopropyl)-1-(4-fluorophenyl)-1,3-dihydroisobenzofuran-5-carbonitrile as a syrup (3.0 g).
   [∝]²⁵_{D} = (+) 12.04° (concentration of 1%, in methanol), chiral purity by HPLC = 98.32%.

### EXAMPLE 4

The compound (-)-1-(3-aminopropyl)-1-(4-fluorophenyl)-1,3-dihydroisobenzofuran-5-carbonitrile (Formula XII) was prepared, as follows:
1-((3-aminopropyl)-1-(4-fluorophenyl)-1,3-dihydroisobenzofuran-5-carbonitrile (10 g) was dissolved in 50 ml of acetonitrile. A solution of (+)-di-p-toluoyltartaric acid (13 g in 20 ml of acetonitrile) was added slowly at ambient temperature. The reaction mixture was stirred at ambient temperature to obtain thick solid. The reaction mixture was heated to 60 -65°C for 45 to 60 minutes and the resulting the reaction mass was cooled to 0-5°C. The reaction mass was stirred at 0-5°C for 1 hour and the resulting solid was filtered under vacuum.and dried at 60-65°C for 6 to hours. The solid was then suspended in 150ml of acetonitrile and heated to reflux. Water (10 ml) was added under reflux to obtain a clear solution. The reaction mixture was cooled to 0-5°C for 3 hours. The precipitated solid was filtered off and suspended in 100 ml of water, pH of the suspension was adjusted to 12, and then it was extracted with toluene (3 x 100 ml). The combined toluene layer was washed with water (2 x 50 ml), dried over anhydrous sodium sulfate, and distilled to afford (-)-1-(3-aminopropyl)-1-(4-fluorophenyl)-1,3-dihydroisobenzofuran-5-carbonitrile as a syrup (2.8 g).
   [∝]²⁵_{D} = (-) 12.07° (concentration 1%, in methanol), chiral purity by HPLC = 98.61%.

### EXAMPLE 5

The compound (+)-1-(3-dimethylaminopropyl)-1-(4-fluorophenyl)-1,3-dihydroisobenzofuran-5-carbonitrile (Formula I) was prepared, as follows:
A solution of (+)-1-(3-aminopropyl)-1-(4-fluorophenyl)-1,3-dihydroisobenzofuran-5-carbonitrile (5 g, 0.017 mole) and formaldehyde (2.5 g, 0.084 mole, 37% aqueous solution) in 98% formic acid (3.88 g, 0.084 mole) was refluxed at 95-100°C for 12 hours. After the solution was cooled, 5 ml of 4N hydrochloric acid solution was added and the resulting reaction solution was evaporated to dryness under reduced pressure. Then 1 N sodium hydroxide solution (100ml) was added to the residue and extracted with diethyl ether (3 x 100 ml). The organic extract was washed with water (2 x 100 ml), dried over anhydrous sodium sulfate and distilled off to afford the compound of formula I in the form of a syrup (4.2 g).
   [∝]²⁵_{D} = (+) 11.26° (concentration 1%, in methanol), chiral purity by HPLC = 97.05%.

### EXAMPLE 6

The compound (-)-1-(3-dimethylaminopropyl)-1-(4-fluorophenyl)-1,3-dihydroisobenzofuran-5-carbonitrile (Formula V) was prepared, as follows:
A solution of (-)-1-(3-aminopropyl)-1-(4-fluorophenyl)-1,3-dihydroisobenzofuran-5-carbonitrile (5 g, 0.017 mole) and formaldehyde (2.5 g, 0.084 mole, 37% aqueous solution) in 98% formic acid (3.88 g, 0.084 mole) was refluxed at 95-100°C for 12 hours. After the solution was cooled, 5 ml of 4N hydrochloric acid solution were added and the resulting reaction solution was evaporated to dryness under reduced pressure. Then 1 N sodium hydroxide solution (100 ml) was added to the residue and extracted with diethyl ether (3 x 100 ml). The organic extract was washed with water (2 x 100 ml), dried over anhydrous sodium sulfate and distilled off to afford the compound R-citalopram in the form of syrup (4.3 g).
   [∝]²⁵_{D} = (-) 12.30° (concentration 1%, in methanol), chiral purity by HPLC = 98.05%.

### EXAMPLE 7

The compound 1-(3-dimethylaminopropyl)-1-(4-fluorophenyl)-1,3-dihydroisobenzofuran-5-carbonitrile (Formula VIII) was prepared, as follows:
A solution of 1-(3-aminopropyl)-1-(4-fluorophenyl)-1,3-dihydroisobenzofuran-5-carbonitrile (5 g, 0.017 mole) and formaldehyde (2.5 g, 0.084 mole, 37% aqueous solution) in 98% formic acid (3.88 g, 0.084 mole) was refluxed at 95-100°C for 12 hours. After the solution was cooled, 5 ml of 4N hydrochloric acid solution was added and the resulting reaction solution was evaporated to dryness under reduced pressure. Then 1 N sodium hydroxide solution (100ml) was added to the residue and extracted with diethyl ether (3 x 100 ml). The organic extract was washed with water (2 x 100 ml), dried over anhydrous sodium sulfate and distilled off to afford the compound citalopram in the form of syrup (4.1 g).

### EXAMPLE 8

The compound 1-(3-methylaminopropyl)-1-(4-fluorophenyl)-1,3-dihydroisobenzofuran-5-carbonitrile (Formula XIII) was prepared, as follows:
A 10 gram portion of 1-(3-aminopropyl)-1-(4-fluorophenyl)-1,3 dihydroisobenzofuran-5-carbonitrile (0.033 mole) was added to benzaldehyde (4.2 g, 0.040 mole) and refluxed in a water separator for 1.5-2 hours in 20 ml of toluene. To the resulting solution was added 5.1 g dimethyl sulfate (0.0405 mole) in 20 ml toluene at such a rate as to maintain reflux (over 15 minutes). The reaction mixture was refluxed for 90 minutes, cooled and slowly treated with 30 ml of water and heated to reflux for an additional 20 minutes. After cooling, the aqueous layer was separated, washed twice with 2 x 15 ml of diethyl ether to remove unreacted benzaldehyde and adjusted to pH 12-13 with 50 % aqueous NaOH. This basic aqueous layer was extracted with 2 x 30 ml of diethyl ether. The organic extract was washed with water (2 x 10 ml), dried over anhydrous sodium sulfate and distilled off to afford 8 g of the compound desmethyl citalopram in the form of a syrup. The oxalate salt of the desmethyl citalopram was crystallized from acetone (8.0 g yield).

### EXAMPLE 9

The compound (+)-1-(3-methylaminopropyl)-1-(4-fluorophenyl)-1,3-dihydroisobenzofuran-5-carbonitrile (Formula IX) was prepared, as follows:
A 10 gram portion of (+)-1-(3-aminopropyl)-1-(4-fluorophenyl)-1,3-dihydroisobenzofuran-5-carbonitrile (0.033 mole) and benzaldehyde (4.2gm, 0.040 mole) were refluxed in a water separator for 1.5-2 hours in 20 ml of toluene. To the resulting solution was added 5.1 g dimethyl sulfate (0.0405 mole) in 20 ml toluene at such a rate as to maintain reflux (over 15 minutes). The reaction mixture was refluxed for 50 minutes, cooled and slowly treated with 30 ml water and heated for an additional 20 minutes. After cooling in ice, the aqueous layer was washed twice with 2 x 15 ml of diethyl ether to remove unreacted benzaldehyde and adjusted to pH 12-13 with 50 % aqueous NaOH. This basic aqueous layer was extracted with 2 x 30 ml of diethyl ether. The organic extract was washed with water (2 x 10 ml), dried over anhydrous sodium sulfate and distilled off to afford 7 g of the compound (+)-desmethylcitalopram in the form of an oil. The oxalate salt of the (+)-desmethyl citalopram was crystallized from acetone. [∝]²⁵_{D} = (+) 9.8° (concentration 1%, in methanol).

### EXAMPLE 10

The compound (-)-1-(3-methylaminopropyl)-1-(4-fluorophenyl)-1,3-dihydroisobenzofuran-5-carbonitrile (Formula X) was prepared, as follows:
10 grams of (-)-1-(3-aminopropyl)-1-(4-fluorophenyl)-1,3-dihydroisobenzofuran-5-carbonitrile (0.033 mole) and benzaldehyde (4.2g, 0.040 mole) were refluxed in a water separator for 1.5-2 hours in 20 ml of toluene. To the resulting solution was added 5.1 g dimethyl sulfate (0.0405 mole) in 20 ml toluene at such a rate as to maintain reflux (over 15 minutes). The reaction mixture was refluxed for 50 minutes, cooled and slowly treated with 30 ml water and heated for an additional 20 minutes. After cooling in ice, the aqueous layer was washed twice with 2 x 15 ml of diethyl ether to remove unreacted benzaldehyde and adjusted to pH 12-13 with 50 % aqueous NaOH. This basic aqueous layer was extracted with 2 x 30 ml of diethyl ether. The organic extract was washed with water (2 x 10 ml), dried over anhydrous sodium sulfate and distilled off to afford 8.2 g of the compound (-)-desmethylcitalopram in the form of an oil. The oxalate salt of the (-)-desmethylcitalopram was crystallized from acetone.

### EXAMPLE 11 (not part of invention)

The compound (+)-1-(3-dimethylaminopropyl)-1-(4-fluorophenyl)-1,3-dihydroisobenzofuran-5-carbonitrile (Formula I) was prepared, as follows:
Racemic 1-(3-dimethylaminopropyl)-1-(4-flourophenyl)-1,3-dihydroisobenzofuran-5-carbonitrile (50g) and (+)-di-p-toluoyl tartaric acid (62g) were suspended in 500 ml of acetonitrile. The reaction mixture was stirred at ambient temperature for a period of 10-15 minutes, then heated to 70-75°C. Methanol (40 ml) was added and a clear solution was obtained. The resulting reaction mass was stirred at the same temperature for a period of 30-45 minutes. Then the reaction mass was cooled to 30-35°C for a period of 2-3 hours and the resulting solid was filtered under vacuum. The filtrate was evaporated to dryness and the resultant thick residue was suspended in aqueous sodium hydroxide solution (1.6 g NaOH in 100 ml water). To the reaction solution, toluene (100ml) was added. The reaction mixture was stirred for a period of 30-45 minutes, the toluene layer was separated and the aqueous basic solution was extracted with toluene (3 x 20ml). The combined toluene layer was washed with water (3 x 20 ml), and toluene was removed by distillation to afford (+)-1-(3-dimethylaminopropyl)-1-(4-fluorophenyl)-1,3-dihydroisobenzofuran-5-carbonitrile as a thick syrup (12.0 g). Then the obtained thick syrup was again subjected to the above process to improve the chiral purity. [∝]²⁵_{D} = (+)10.8° (concentration 1 %, in methanol), chiral purity by HPLC = 98.89%.

### EXAMPLE 12

The following procedures were used to prepare the crystalline oxalate salt of (+)-1-[(3-dimethylamino)propyl]-1-(4-fluorophenyl)-1,3-dihydro-5-isobenzofuran carbonitrile (escitalopram oxalate):

### Method 1

35 g of the (-)-di-p-toluoyl tartaric acid salt of 1-(3-aminopropyl)-1-(4-fluorophenyl)-1,3-dihydro-5-isobenzofurancarbonitrile, 140 ml of water and 140 ml of toluene were combined with stirring followed by adjusting the pH to 11-13 with 10.5 ml of 10% NaOH. The aqueous layer was separated and extracted with toluene (2 x 70 ml). The combined organic layer was washed with water (3 x 87.5 ml) at 65-70°C, then the solvent was evaporated under reduced pressure to obtain an oil material. 140 ml of water, 6.4 ml of 98% formic acid and 12.7 ml of 37% formaldehyde were added to the oil and heated to 95-100°C for about 3-4 hours. The reaction mass was washed with toluene (2 x 35 ml). The aqueous layer pH was adjusted to 1-2 with 5.25 ml of hydrochloric acid and washed with methyl tertiary-butyl ether (2 x 35 ml). The aqueous layer pH was adjusted to 9-10 with 10.5 ml of 10% NaOH and extracted with toluene (3 x 35 ml). The combined organic layer was washed with water (3 x 35 ml) at 65-70°C. Solvent was evaporated under reduced pressure to produce an oil. The oil was dissolved in 52.6 ml of ethyl acetate at 30-35°C and filtered to remove particles. The solution was added slowly to an oxalic acid solution (5.4 g of oxalic acid in 120 ml of ethyl acetate, dissolved at 40-45°C and filtered) over about 25-30 minutes at 25-30°C, heated to 65-70°C for about 35-45 minutes, and then cooled to 0-5°C for about 1-2 hours. The solid that formed was filtered and washed with 15 ml of ethyl acetate, and dissolved by heating with 90 ml of ethyl acetate at 65-70°C for 30-40 minutes. After cooling to 0-5°C for about 1-2 hours, the solid was filtered and washed with 15 ml of ethyl acetate. The solid was dried at 55-60°C for about 6-8 hours, resulting in the desired crystalline form of the compound (yield: 14.9 g).

The obtained crystalline escitalopram oxalate had a particle size distribution, as measured by a Malvern particle size analyzer, as follows: 90% less than 40 *µ*m, 50% less than 13 *µ*m, and 10% less than 4 *µ*m.

### Method 2

5 grams of escitalopram oxalate were dissolved in 25 ml isopropyl alcohol, by heating at 50-60°C for 30-40 minutes. After cooling to 0-5°C for about 1-2 hours, the solid that formed was filtered and washed with 10 ml of isopropyl alcohol. The solid was dried at 55-60°C for about 6-8 hours to obtain the desired crystalline form (yield: 4.5 g).

The crystalline of escitalopram oxalate can also be prepared using this general crystallization method with other organic solvents such as ethyl acetate, methanol, ethanol, 2-butanol, acetonitrile, petroleum ether, acetone, water, and mixtures thereof.

### EXAMPLE 13 (not part of the invention)

The following procedure was used to prepare the L-(-)-DPTTA salt of 1-(3-aminopropyl)-1-(4-fluorophenyl)-1,3-dihydroisobenzofuran-5-carbonitrile:

### Step A:

47 g of 3-chloropropyl amine, 94 ml of water, 15.9 g of sodium hydroxide and 265 ml of toluene were combined and stirred at 25-35°C for 35-40 minutes. The aqueous layer was separated and extracted with toluene (2 x 117.5 ml), then the organic layers were combined for use in the following step.

### Step B:

A solution was prepared by adding 35.5 g of potassium tertiary-butoxide to DMSO (250 ml) at 60-65°C under nitrogen atmosphere. To the resulting solution a solution of 1-(4-fluorophenyl)-1,3-dihydroisobenzofuran-5-carbonitrile (50 g) in DMSO (150 ml) was added over 10 minutes at 25 -35 °C. After maintaining at this temperature for 15-20 minutes, the solution of 3-chloropropyl amine from step A was added at once. After the addition, the reaction mixture was heated slowly to 40-45°C and maintained for 60 to 70 minutes. The reaction mixture was then quenched with ice-cold water (500 ml) and the aqueous layer was separated and extracted with toluene (2 x 250 ml). 500 ml of water were added to the combined toluene layers and the pH was adjusted to less than 2 using 24 ml of hydrochloric acid. The aqueous layer was separated and washed with toluene (3 x 250 ml). The aqueous layer pH was adjusted to 10-12 with an NaOH solution and extracted with toluene (3 x 250 ml). The combined organic layers were washed with water (3 x 250 ml) at 60-65°C and solvent was removed by distillation at reduced pressure below 65°C, resulting in a thick syrup.

The obtained syrup was dissolved in 400 ml of acetonitrile and a solution of 52.1 grams of (-)-di-*p*-toluoyl tartaric acid ("DPTTA") in 106 ml of acetonitrile was added at 20-30°C for 10-15 minutes. 95 ml of water were added at 55-60°C and the mixture was stirred for 45 minutes. After cooling to 0-5°C for about 2-3 hours, the formed solid was separated by filtration and washed with 42 ml of acetonitrile. The solid was dried at 60-65°C for 6-8 hours.

### EXAMPLE 14

The DPTTA salt of 1-(3-aminopropyl)-1-(4-fluorophenyl)-1,3-dihydroisobenzofuran-5-carbonitrile was purified using the following procedure:
100 grams of the DPTTA salt of 1-(3-aminopropyl)-1-(4-fluorophenyl)-1,3-dihydroisobenzofuran-5-carbonitrile and 110 ml of acetonitrile were heated to 45-55°C, then 300 ml of water were added and the mixture stirred for 1.5 hours at 45-55°C. The mixture was cooled to 0-5°C and stirred for 2 hours, then the temperature was raised to 25-30°C and the mixture stirred for 45 minutes; optionally repeating this process two or three times. The solid was filtered and washed with 200 ml of chilled acetonitrile, then dried at 60-65°C for 6-8 hours. 85 grams of the dried solid were added to 850 ml of acetonitrile, heated to 45-55°C and 250 ml of water were added, followed by stirring for 1.5 hours at 45-55°C. The mixture was cooled to 0-5°C and stirred for another 2 hours. The formed solid was separated by filtration and washed with 170 ml of chilled acetonitrile, then was dried at 60-65°C for 6-8 hours.

### EXAMPLE 15

When escitalopram is prepared by a process that includes methylation of an amino group using formaldehyde and formic acid, as was discussed above, variable amounts of an N-formyl impurity are usually formed. Generally, this impurity will be present in the escitalopram in amounts less than about 0.2 percent by weight, typically less than about 0.1 percent, preferably less than about 0.01 percent, and more preferably less than about 0.001 percent. For purposes of characterizing the impurity, the compound (N-{3-[5-cyano-1-(4-fluorophenyl)-1,3-dihydro-2-benzofuran-1-yl] propyl} formamide) has been prepared by the following procedure:
10 grams of 1-(3-aminopropyl)-1-(4-fluorophenyl)-1,3-dihydroisobenzofuran-5-carbonitrile, 16.5 ml of formic acid, and 100 ml of toluene were heated to 95-100°C for 6-8 hours under refluxing conditions. Then, 100 ml of water were added to the reaction mass at 25-30°C and the mixture was stirred for 15 minutes. The organic layer was separated and washed with water (2 x 100 ml). Solvent was distilled from the organic layer under reduced pressure between 40 and 60°C and the solid compound was isolated. This compound is the N-formyl impurity of escitalopram, having the structure given below and the following spectral data: ¹H-NMR (200 MHz) in DMSO-D₆, ppm: 1.2 (m, 2H), 2.2 (m, 2H), 3.2 (q, 2H), 5.2 (q, 2H), 7-7.8 (m, 7H), 8.0 (s, 1 H, -CHO);

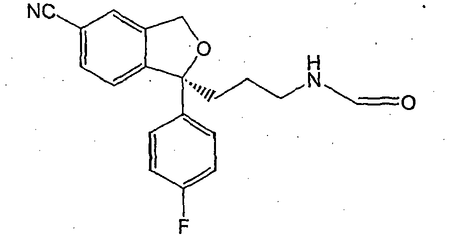

The amount of this N-formyl impurity can be minimized by making use of at least one of these general principles, in the synthesis of escitalopram oxalate:
(i) use a stoichiometric excess of formic acid, such as was shown in Example 7;
(ii) since the N-formyl impurity cannot convert to an acid salt at low pH values, it can be separated in an acidified environment by solvent washings, such as with toluene or ether washings;
(iii) the N-formyl impurity is soluble in certain solvents such as ethyl acetate, and can be selectively washed out at the end of the procedure for forming the oxalate.

## Claims

1. A process for preparing escitalopram having the structure: 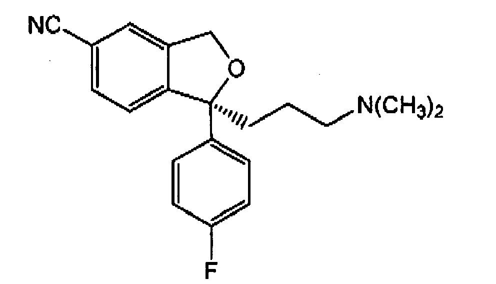 comprising the steps of:
(a) reacting 5-cyano-1-(4-fluorophenyl)-1-aminopropyl-1,3-dihydroisobenzofuran having the structure: 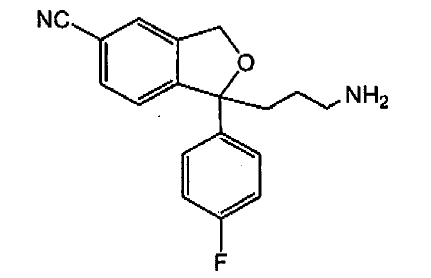 with an enantiomerically pure acid;
(b) hydrolyzing a product from (a) using a base;
(c) methylating a product recovered from (b); and
(d) recovering escitalopram.

2. A process according to claim 1 wherein an enantiomerically pure acid is a dibenzoyltartaric acid, a di-p-toluoyl tartaric acid, an o-nitrobenzoyl tartaric acid, lactic acid, bisnaphthylphosphoric acid, 10-camphorsulfonic acid, 8-camphorsulfonic acid, malic acid, N-acetyl glutamic acid, or mandelic acid.

3. A process according to any preceding claim wherein an enantiomerically pure acid is (-)-di-p-toluoyl tartaric acid.

4. A process according to claim 1, wherein a product from (b) is methylated using methyl iodide, dimethyl sulfate or a mixture of formic acid and formaldehyde.

5. A process according to claim 1 wherein recovered escitalopram contains less than 0.2 weight percent of N-{3-[5-cyano-1-(4-fluorophenyl)-1,3-dihydro-2-benzofuran-1-yl] propyl} formamide.

6. A process according to claim 1 wherein recovered escitalopram contains less than 0.01 weight percent of N-{3-[5-cyano-1-(4-fluorophetlyl)-1,3-dihydro-2-benzofuran-1-yl] propyl} formamide.

## Patentansprüche

1. Verfahren zur Herstellung von Escitalopram mit der Struktur 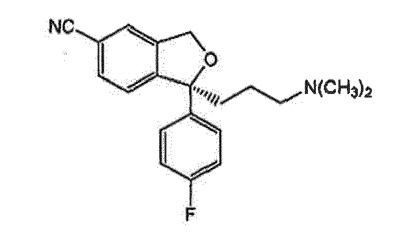 das die folgenden Schritte beinhaltet:
(a) Umsetzen von 5-Cyano-1-(4-fluorphenyl)-1-aminopropyl-1,3-dihydroisobenzofuran mit der Struktur: 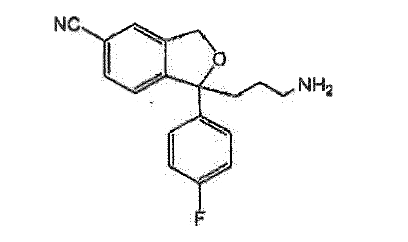 mit einer enantiomer reinen Säure;
(b) Hydrolysieren eines Produkts von (a) mit einer Base;
(c) Methylieren eines aus (b) gewonnenen Produkts; und
(d) Gewinnen von Escitalopram.

2. Verfahren nach Anspruch 1, wobei eine enantiomer reine Säure eine Di-benzoylweinsäure, eine Di-p-toluoylweinsäure, eine o-Nitrobenzoylweinsäure, Milchsäure, Bisnaphthylphosphorsäure, 10-Camphersulfonsäure, 8-Camphersulfonsäure, Apfelsäure, N-Acetylglutaminsäure oder Mandelsäure ist.

3. Verfahren nach einem der vorherigen Ansprüche, wobei eine enantiomer reine Säure (-)-Di-p-toluoylweinsäure ist.

4. Verfahren nach Anspruch 1, wobei ein Produkt aus (b) mit Methyliodid, Dimethylsulfat oder einem Gemisch aus Ameisensäure und Formaldehyd methyliert wird.

5. Verfahren nach Anspruch 1, wobei gewonnenes Escitalopram weniger als 0,2 Gew.-% N-{3-[5-Cyano-1-(4-fluorphenyl)-1,3-dihydro-2-benzofuran-1-yl]propyl}formamid enthält.

6. Verfahren nach Anspruch 1, wobei gewonnenes Escitalopram weniger als 0,01 Gew.-% N-{3-[5-Cyano-1-(4-fluorphenyl)-1,3-dihydro-2-benzofuran-1-yl]propyl}formamid enthält.

## Revendications

1. Procédé de préparation d'escitalopram ayant la structure : 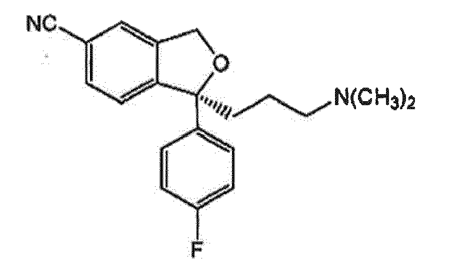 comprenant les étapes consistant à :
(a) faire réagir du 5-cyano-1-(4-fluorophényl)-1-aminopropyl-1,3-dihydroisobenzofurane ayant la structure : 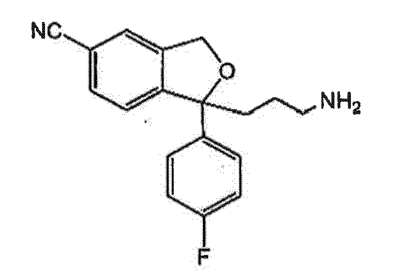 avec un acide énantiomériquement pur;
(b) hydrolyser un produit de (a) avec une base;
(c) méthyler un produit récupéré de (b); et
(d) récupérer l'escitalopram.

2. Procédé selon la revendication 1, dans lequel l'acide énantiomériquement pur est un acide dibenzoyltartrique, un acide di-p-toluoyltartrique, un acide o-nitrobenzoyltartrique, un acide lactique, un acide bisnaphtylphosphorique, un acide 10-camphosulfonique, un acide 8-camphosulfonique, un acide malique, un acide N-acétylglutamique ou un acide mandélique.

3. Procédé selon l'une quelconque des revendications précédentes, dans lequel l'acide énantiomériquement pur est de l'acide (-)-di-p-toluoyltartrique.

4. Procédé selon la revendication 1, dans lequel un produit de (b) est méthylé en utilisant de l'iodure de méthyle, du sulfate de diméthyle ou un mélange d'acide formique et de formaldéhyde.

5. Procédé selon la revendication 1, dans lequel l'escitalopram récupéré contient moins de 0,2 pour cent en poids de N-{3-[5-cyano-1-(4-fluorophényl)-1,3-dihydro-2-benzofuran-1-yl]propyl}formamide.

6. Procédé selon la revendication 1, dans lequel l'escitalopram récupéré contient moins de 0,01 pour cent en poids de N-{3-[5-cyano-1-(4-fluorophényl)-1,3-dihydro-2-benzofuran-1-yl]propyl}formamide.
